# EUROPÄISCHE PATENTSCHRIFT

(11) **EP 0 951 868 B1**
(45) Veröffentlichungstag und Bekanntmachung des Hinweises auf die Patenterteilung: **25.06.2003**
(21) Anmeldenummer: 98107644.1
(22) Anmeldetag: 27.04.1998
(51) Int. Cl.: A61B 17/02

(54) **Chirurgischer Spreizer**
Surgical retractor
Ecarteur chirurgical

(43) Veröffentlichungstag der Anmeldung: 27.10.1999
(73) Patentinhaber: Waldemar Link (GmbH & Co.), 22339 Hamburg (DE)
(72) Erfinder: Büttner-Janz, Karin, Dr., 12621 Berlin (DE); Keller, Arnold, 23863 Kayhude (DE)
(74) Vertreter: Glawe, Delfs, Moll & Partner

(56) Entgegenhaltungen:
- EP-A- 0 792 620
- FR-A- 1 005 345
- US-A- 2 693 795
- US-A- 5 167 662
- US-A- 5 303 694
- US-A- 5 529 571

## Beschreibung

Es sind Wundspreizer mit parallel geführten Valven bekannt, bei denen jede Valve an einer von zwei Halteschienen angeordnet ist, von denen eine fest und die andere verschiebbar mit einer Querführungsstange verbunden ist. Die Verstellung eines solchen Spreizers ist weniger einfach als die eines anderen bekannten Wundspreizertyps, bei dem die Valven an den Enden zweier scherenförmig zueinander geführter Glieder sitzen, deren andere Enden Ringgriffe tragen.

Der Erfindung liegt die Aufgabe zugrunde, die Vorteile der Parallelführung mit denen der leichteren Betätigung zu verbinden.

Die erfindungsgemäße Lösung besteht in den Merkmalen des Anspruchs 1 und vorzugsweise denen der Unteransprüche. Danach ist ein Spreizer mit *Valven, die an Halteschienen angeordnet sind, die mit einer Parallelführung und* einer *Rückstellsperre versehen sind,* vorgesehen, der mit einem scherenförmigen Betätigungsinstrument verbunden bzw. verbindbar ist. *Dazu weisen das* Betätigungsinstrument und die Halteschienen oder deren Parallelführungen weisen *im Spreizzustand* lösbar miteinander verbundene bzw. verbindbare Kupplungen auf. Das hat den Vorteil, daß das Betätigungsinstrument eine bequeme Handhabung ermöglicht wie bei bekannten scherenförmigen Spreizern, aber *das Operationsfeld* *von dem nach der Einstellung des Parallelspreizers unnötigen Betätigungsinstrument befreit werden kann. Dadurch erhält der Operateur mehr Platz, um insbesondere bei dorsalen Operationen der Lendenwirbelsäule direkt über dem Operationsfeld in die Tiefe der Wunde schauen zu können.*

*Es ist zwar ein Spreizinstrument bekannt (US-A-5 529 571), das einen Spreizteil mit an Halteschienen angeordneten Valven und einen gelenkig mit den Enden der Halteschienen verbundenen Betätigungsteil aufweist, jedoch bilden bei dem Instrument Spreizteil und Betätigungsteil eine Einheit, die im gespreizten Zustand nicht getrennt werden kann. Die Rückstellsperre ist nämlich am Betätigungsteil angeordnet ist und muß daher an dem Spreizteil verbleiben.*

Nach einem besonderen Merkmal der Erfindung, das gegebenenfalls von den vorgenannten Merkmalen unabhängigen Schutz verdient, ist die Parallelführung der Halteschienen als Scherenanordnung ausgebildet. Dies gestattet eine besonders reibungsarme und daher bequeme Verstellung. Ein besonderer Vorteil dieser Anordnung besteht darin, daß der Kreuzungspunkt der Scherenanordnung zur Befestigung einer mittleren Valvenhalteschiene vorgesehen werden kann, die dann zwangsläufig mittig zu den an den beiden äußeren Halteschienen angeordneten Valven gehalten ist. Diese mittlere Valvenhalteschiene kann mit einer weiteren Betätigungskupplung zur Verbindung mit einem Glied des Betätigungsinstruments versehen sein, dessen anderes Glied eine Valve oder Valvenkupplungen trägt. Statt dessen oder zusätzlich könnte eine geeignete Spreizzange mit einem Arm angebracht werden, deren anderer Arm eine Valve hält. Dadurch wird die Möglichkeit geschaffen, den Spreizer außer mit den von den beiden äußeren Valvenhalteschienen gehaltenen Valven mit einem quer dazu wirkenden weiteren Valvenpaar zu versehen. Dadurch wird es möglich, mit einem und demselben Instrument eine Wunde nach allen vier Richtungen hin zu spreizen.

Damit die am Betätigungsinstrument vorgesehenen Betätigungskupplungen sowohl zur Verbindung mit der mittleren Valvenschiene als auch mit einer Valve dienen können, sind die Betätigungskupplungen einerseits und die Valvenkupplungen andererseits zweckmäßigerweise übereinstimmend ausgebildet.

*Zwar ist es ausreichend,* ausschließlich den Parallelspreizer mit einer Rückstellsperre zu versehen-; wenn jedoch, wie oben erläutert, das Betätigungsinstrument auch noch als zusätzlicher Spreizer zur Ergänzung des Parallelspreizers in anderer Spreizrichtung verwendet wird, wird auch das Betätigungsinstrument mit einer Rückstellsperre versehen.

Nach einem weiteren Merkmal der Erfindung, das gegebenenfalls Schutz unabhängig von den vorgenannten Merkmalen verdient, sind die parallel geführten Halteschienen je mit einer Reihe von Valvenkupplungen ausgerüstet. Dies gestattet es, statt der üblichen einen Valve deren mehrere an jeder Seite vorzusehen, was eine sicherere und schonendere Offenhaltung der Wunde ermöglicht. Auch gibt dieses Merkmal die Möglichkeit, außer einer oder mehreren Valven auch noch irgendwelche Zusatzinstrumente, beispielsweise eine Lichtquelle, daran zu positionieren. Zu diesem Zweck können die Zusatzinstrumente mit zu den Valvenkupplungen der Halteschienen passenden Kupplungen versehen sein.

Das Merkmal, daß das Betätigungsinstrument scherenförmig ist, besagt nicht, daß die beiden gelenkig miteinander verbundenen Scherenglieder einander im Gelenkpunkt überkreuzen müssen. Vielmehr ist es zweckmäßig, daß sie derart miteinander verbunden sind, daß beim Zusammendrücken ihrer Griffenden ihre anderen Enden, die mit dem Spreizer gekuppelt sind, sich voneinander entfernen. Meist wird der Spreizer in Verbindung mit Valven verwendet. Jedoch können statt der Valven von üblicher Form auch andere Greifelemente daran vorgesehen werden. Beispielsweise können die Halteschienen statt der zur Verbindung mit Valven vorgesehenen Kupplungselemente oder zusätzlich zu diesen mit Bohrungen zum Einbringen von Kirschnerdrähten versehen sein. So kann das Instrument auch zum Spreizen von Knochen verwendet werden, in die ein Kirschnerdraht oder mehrere Kirschnerdrähte eingebohrt oder eingeschlagen werden. Die Bohrungen können so angeordnet werden, daß je ein Kirschnerdraht wahlweise ohne oder in Kombination mit einer Valve verwendet werden kann, wobei er entsprechend in der Valve vorgesehene Bohrungen durchdringt. Dadurch wird zum einen die Valve gegenüber der Halteschiene fixiert, d.h. sie kann sich gegenüber der Schiene nicht verdrehen. Zum anderen wird der von der Valve erfaßte Körperteil, also beispielsweise ein Knochen, an der Valve fixiert. Der Kirschnerdraht kann auch alleine durch die Valvenbohrungen geführt werden. Dadurch wird die Valve fixiert, bleibt aber drehfähig.

Die Erfindung wird im folgenden näher unter Bezugnahme auf das in der Zeichnung dargestellte Ausführungsbeispiel beschrieben. Darin zeigen:
- Fig. 1: eine perspektivische Gesamtansicht mit zum Spreizen des Parallelspreizers angesetztem Betätigungsinstrument,
- Fig. 2: eine Draufsicht auf den gemäß Fig. 1 eingestellten Parallelspreizer ohne Betätigungsinstrument,
- Fig. 3: eine Draufsicht auf das Instrument mit als zusätzlichem Scherenspreizer angesetztem Betätigungsinstrument,
- Fig. 4: eine perspektivische Gesamtansicht der Anordnung gemäß Fig. 3 mit zusätzlicher Lichtquelle und
- Fig. 5: eine Detailansicht einer Ausführungsvariante.

Der Parallelspreizer 1 umfaßt zwei Halteschienen 2, die an einer aus zwei Gliedern 3 gebildeten Parallelführungsschere angeordnet sind. Einerseits sind die Enden der Halteschienen im Gelenkpunkt 4 nahe einem Ende der Glieder 3 mit diesen gelenkig verbunden. Andererseits gleitet das am anderen Ende der Glieder 3 gebildete Gelenk 5 in einem Langloch 6 der Halteschienen 2, wobei die Gelenkpunkte 4 und 5 jeweils gleichen Abstand vom Kreuzungspunkt 7 der Scherenglieder 3 haben.

Die über die Scherenanordnung 3 bis 7 hinaus auskragenden Enden der Halteschienen 2 enthalten eine Reihe von Bohrungen 10 als Kupplungen für die wahlfreie Verbindung mit komplementär an den Valven 11 vorgesehenen Kupplungen, die als in die Bohrungen 10 mit Gleitsitz passende Stifte 19 ausgeführt sind. Innerhalb der Bohrungen 10 sind Federkugelhalterungen 12 angeordnet, deren Kugeln unter Federkraft in nicht dargestellten Ringnuten der an den Valven 11 vorgesehenen Kupplungsstifte eingreifen, um diese hinreichend fest, aber lösbar in den Bohrungen 10 festzuhalten.

Die Bohrungen 10 sind ferner mit Längsnuten 13 versehen, die mit an den Kupplungsstiften 19 vorgesehenen, seitlichen Vorsprüngen zusammenwirken können, falls undrehbare Verbindung der angesetzten Instrumente mit den Halteschienen gewünscht ist. In der Regel sind aber die Kupplungsstifte 19 der Valven 11 ohne solche Vorsprünge ausgeführt, um eine geeignete Drehstellung einnehmen zu können, die gegebenenfalls von der Richtung der Halteschienen 2 abweicht.

Am Kreuzungspunkt 7 der von den Gliedern 3 gebildeten Scherenanordnung ist mittels einer Feststellschraube 15 eine mittlere Valvenhalteschiene 16 lösbar angebracht, die am Ende mit einer Kupplungsbohrung 10 zum Halten einer in einer Mittelebene wirkenden Valve 17 ausgerüstet ist. Sie ist über ein Langloch 18 in ihrer Längsrichtung und außerdem im Winkel bezüglich der Achse des Kreuzungspunkts mittels der Feststellschraube 15 verstellbar. Außer der Kupplungseinrichtung 10 zur Anbringung einer Valve 17 ist die mittlere Valvenhalteschiene 16 mit einem Kupplungsstift 19 für später zu erläuternde Zwecke versehen.

Zwischen den Enden 20 der die Scherenanordnung bildenden Glieder 3 ist eine Gewindespindel 21 mit einer Arretiermutter 22 als Rückstellsperre vorgesehen. Die Arretiermutter 22 bestimmt mit ihrer jeweiligen Einstellung den Mindestabstand der Halteschienen 2.

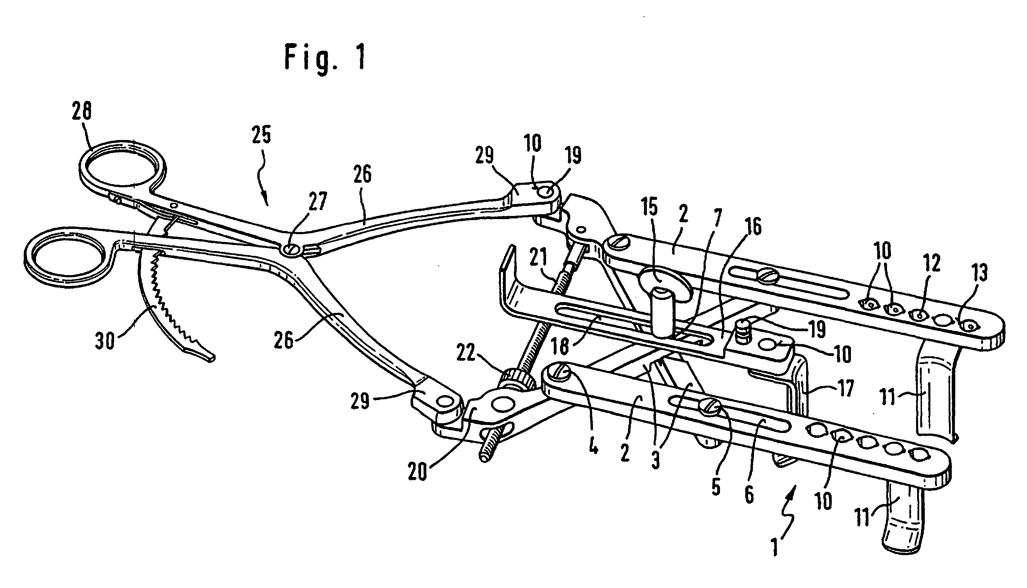

Ebenfalls an den Enden 20 der die Scherenanordnung bildenden Glieder 3 sind Kupplungsstifte 19 vorgesehen, die den Kupplungsstiften an den Valven 11,17 gleichen. Wie Fig. 1 zeigt, dienen sie zur Anbringung des Betätigungsinstruments 25, das in der Art eines Scherenspreizers von zwei Gliedern 26 gebildet ist, die im Gelenkpunkt 27 miteinander verbunden sind und an ihrem freien Ende Ringgriffe 28 tragen. Ihre Kupplungsenden 29 enthalten eine Kupplungsbohrung 10, die den Kupplungsbohrungen 10 in den Valvenhalteschienen gleicht. Die Verbindung 27 der Scherenglieder 26 ist so ausgeführt, daß beim Zusammendrücken der Ringgriffe 28 die Kupplungsenden 29 sich voneinander entfernen. Das Zusammendrücken der Ringgriffe führt damit zu einer Spreizung des Parallelspreizers 1. Die jeweils erreichte Spreizstellung wird zunächst durch eine am Betätigungsinstrument 25 vorgesehene Rückstellsperre 30 verhindert, die in herkömmlicher Weise eine federbelastete Sägezahnstange umfaßt. Nachdem die erreichte Spreizstellung so gesichert ist, kann sie am Parallelspreizer 1 durch die Verschiebung der Arretiermutter 22 in die jeweilige Endstellung gesichert werden. Danach kann das Betätigungsinstrument 25 dank der Lösbarkeit der Kupplung 10/19 vom Parallelspreizer gelöst werden.

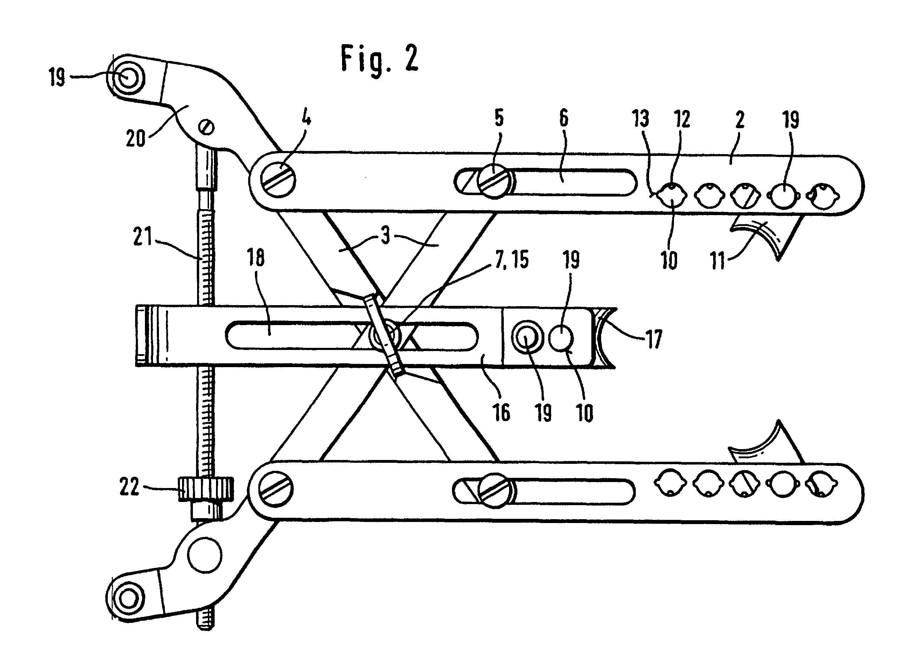

Man erhält dann beispielsweise die in Fig. 2 gezeigte Valvenanordnung. Die an den Halteschienen 2 vorgesehenen Valven 11 bilden mit der an der mittleren Valvenhalteschiene 16 vorgesehenen Valve 17 eine Dreiecksanordnung. Wird die Spreizstellung der Valven 11 nachträglich verändert, behält die mittlere Valve 17 dank ihrer zentralen Anbringung ihre Stellung im Verhältnis zu den beiden anderen Valven 11 im wesentlichen bei. Es ist aber auch möglich, die mittlere Valve 17 unabhängig von den beiden anderen Valven 11 zu verstellen.

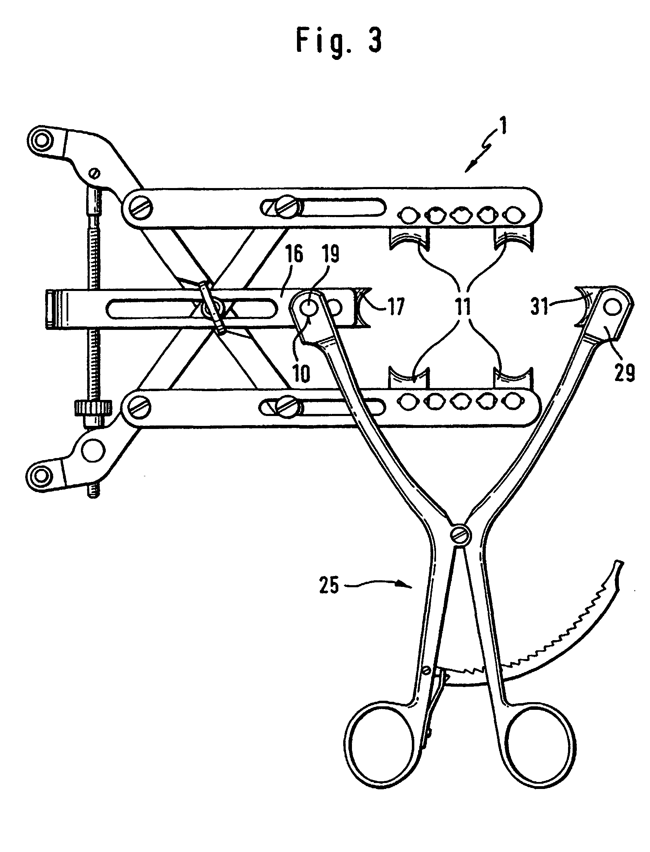

Selbstverständlich kann man die mittlere Valve 17 und die zugehörige Valvenhalteschiene 16 auch weglassen und ausschließlich mit den Valven 11 arbeiten. Dabei gestattet es die Reihenanordnung einer Mehrzahl von Kupplungsbohrungen 10 in den Valvenhalteschienen 2, eine Mehrzahl von parallel geführten Valven vorzusehen, wie dies beispielsweise in Fig. 3 dargestellt ist.

In der Anordnung gemäß Fig. 3 ist das Betätigungsinstrument 25, das hier als Scherenspreizer eingesetzt ist, mit seiner Kupplungsbohrung 10 auf den Kupplungsstift 19 der mittleren Valvenhalteschiene 16 aufgesetzt. Sein anderes Ende 29 trägt eine weitere Valve 31, die der Valve 17 der mittleren Valvenhalteschiene 16 diametral gegenübersteht und zu den von den äußeren Halteschienen gehaltenen Valven 11 beispielsweise mittig (oder auch anders) angeordnet sein kann. Dadurch gelangt man zu einer Valvenkonfiguration, die eine Spreizung in vier verschiedenen Richtungen ermöglicht, wobei in einer Richtung eine Mehrzahl von Valven nebeneinander vorgesehen sein kann, so daß auch große und tiefe Operationsfelder in definierter Weise offengehalten werden können. Das Instrument eignet sich besonders für dorsale Wirbelsäuleneingriffe, insbesondere Nukleotomien, aber auch beispielsweise für ventrale Halswirbelsäulenoperationen und Extremitäteneingriffe.

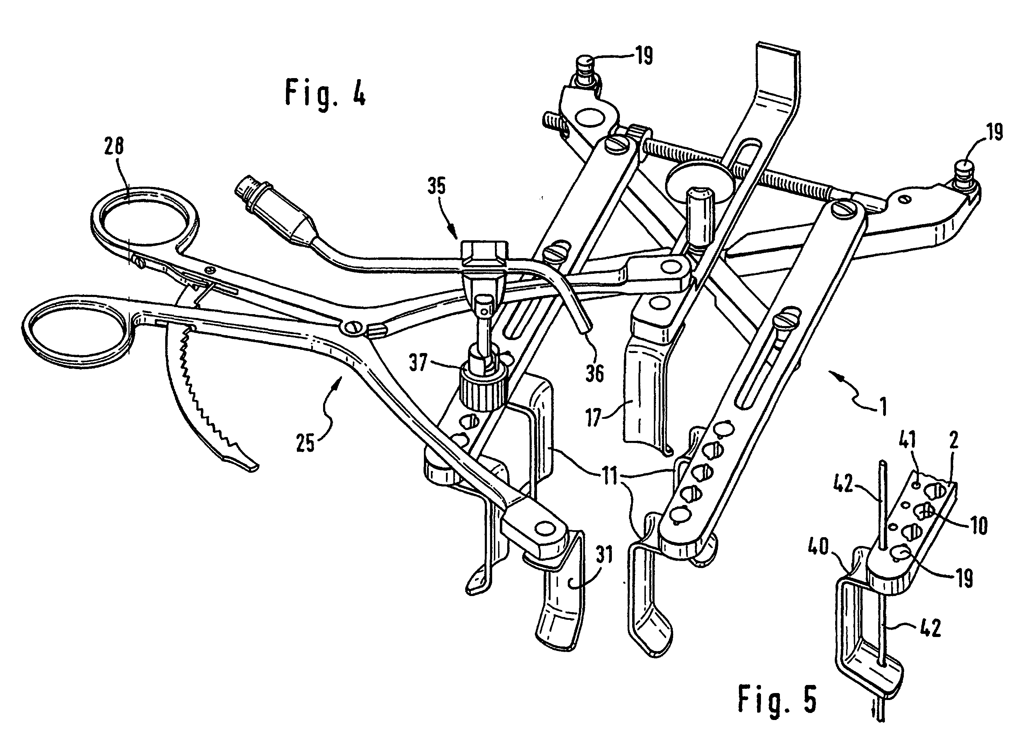

Diese Anordnung ist in Fig. 4 perspektivisch veranschaulicht. Außerdem zeigt diese Darstellung ein Zusatzinstrument 35, das eine Lichtleiter-Lichtquelle 36 und einen damit verbundenen, vielseitig verstellbaren Halter 37 umfaßt und das (in dieser Fig. nicht erkennbar) am unteren Ende einen Kupplungsstift trägt, der den oben beschriebenen Kupplungsstiften 19 gleicht und daher in eine der Bohrungen 10 der Valvenhalteschienen 2 eingesetzt werden kann.

In der in Fig. 5 angedeuteten Ausführungsvariante ist die Halteschiene 2 zusätzlich zu den in diesem Fall ein wenig nach außen gerückten Kupplungsbohrungen 10 für Valven 40 mit Bohrungen 41 versehen, die unmittelbar neben den Kupplungsbohrungen 10 angeordnet sind und deren Weite dem Durchmesser eines Kirschnerdrahts 42 entspricht. Entsprechende Bohrungen sind auch in den Valven 40 vorgesehen. Die Bohrungen 41 gestatten es, Kirschnerdrähte 42 anstelle von Valven 40 zu verwenden. Außerdem besteht die Möglichkeit, eine Valve 40 mit einem Kirschnerdraht 42 gemeinsam anzuwenden, wie es in Fig. 5 angedeutet ist. Der Kirschnerdraht 42 verhindert dann eine Drehung der Valve 40 um die Mittelachse ihres Kupplungsstifts 19 und fixiert sie dadurch im Verhältnis zur Halteschiene 2. Außerdem kann ein von der Valve 40 gehaltener Knochen mittels des Kirschnerdrahts 42 an der Valve 40 fixiert werden. Schließlich gibt die dargestellte Anordnung auch die Möglichkeit, beispielsweise ein Knochenfragment mittels des nach unten über die Valve 40 hinausragenden Endes des Kirschnerdrahts 42 zu fixieren.

## Patentansprüche

1. Chirurgischer Spreizer mit Valven (11), *die an Halteschienen (2) angeordnet sind, die mit einer Parallelführung (3-7) und einer Rückstellsperre (21, 22) versehen sind,* **dadurch gekennzeichnet, daß** der *Spreizer* mit einem scherenförmigen Betätigungsinstrument (25) *über im Spreizzustand lösbare Betätigungskupplungen (10, 19)* verbunden oder verbindbar ist.

2. Spreizer nach Anspruch 1, **dadurch gekennzeichnet, daß** eine Scherenanordnung (3-7) als Parallelführung der Halteschienen (2) vorgesehen ist.

3. Spreizer nach Anspruch 2, **dadurch gekennzeichnet, daß** am Kreuzungspunkt (7) der Scherenanordnung (3-7) eine mittlere Valve (17) oder Valvenhalteschiene (16) angeordnet ist.

4. Spreizer nach Anspruch *3*, **dadurch gekennzeichnet, daß** die mittlere Valvenhalteschiene (16) mit einer Betätigungskupplung (19) zur Verbindung mit einem Glied (3) des Betätigungsinstruments (25) versehen ist und dieses am anderen Glied (3) eine Valve (17) oder eine Valvenkupplung (10) trägt.

5. Spreizer nach einem der Ansprüche 1 bis 4, **dadurch gekennzeichnet, daß** die Betätigungskupplungen (10,19) und Valvenkupplungen (10,19) übereinstimmend ausgeführt sind.

6. Spreizer nach einem der Ansprüche *3* bis *5*, **dadurch gekennzeichnet, *daß*** *außer* der Parallelführung (3-7) auch das Betätigungsinstrument (25) mit *einer* Rückstellsperre (30) versehen *ist*.

7. Spreizer nach einem der Ansprüche 1 bis *6*, **dadurch gekennzeichnet, daß** die parallel geführten Halteschienen (2) je eine Reihe von Valvenkupplungen (10) aufweisen.

8. Spreizer nach Anspruch 7, **dadurch gekennzeichnet, daß** ein oder mehrere Zusatzinstrumente (35) mit zu den Valvenkupplungen (10) der Halteschienen (2) passenden Kupplungen vorgesehen sind.

9. Spreizer nach einem der Ansprüche 1 bis *8*, **dadurch gekennzeichnet, daß** in den Halteschienen (2) und/oder nahe dem Kreuzungspunkt (7) der Scherenanordnung (3-7) und/oder an wenigstens einem Ende (29) des Betätigungsinstruments (25) Bohrungen (41) zur Aufnahme von Kirschnerdrähten (42) vorgesehen sind.

10. Spreizer nach Anspruch 9, **dadurch gekennzeichnet, daß** auch die Bohrungen (41) für die Kirschnerdrähte (42) nahe den Valvenkupplungen (10) angeordnet sind und auch die Valven (11) Bohrungen (41) zur Aufnahme von Kirschnerdrähten (42) enthalten.

## Claims

1. A surgical retractor with blades (11) which are disposed on retaining bars (2) which are provided with a parallel guide (3-7) and a resetting lock (21,22), **characterised in that** the retractor is connected or can be connected to a scissor-like actuating instrument (25) via actuating couplings (10,19) which can be released in the retracted state.

2. A retractor according to Claim 1, **characterised in that** a scissors assembly (3-7) is provided as the parallel guide for the retaining bars (2)

3. A retractor according to Claim 2, **characterised in that** a central blade (17) or blade retaining bar (16) is disposed at the point of intersection (7) of the scissors assembly (3-7).

4. A retractor according to Claim 3, **characterised in that** the central blade retaining bar (16) is provided with an actuating coupling (19) for connection to a member (3) of the actuating instrument (25) and the latter carries on the other member (3) a blade (17) or a blade coupling (10).

5. A retractor according to any one of Claims 1 to 4, **characterised in that** the actuating couplings (10,19) and blade couplings (10,19) are designed to be congruent.

6. A retractor according to any one of Claims 3 to 5, **characterised in that** in addition to the parallel guide the actuating instrument (25) is also provided with a resetting lock (30).

7. A retractor according to any one of Claims 1 to 6, **characterised in that** the parallel-guided retaining bars (2) each have a row of blade couplings (10).

8. A retractor according to Claim 7, **characterised in that** one of more additional instruments (35) are provided with couplings fitting the blade couplings (10) of the retaining bars (2).

9. A retractor according to any one of Claims 1 to 8, **characterised in that** bores (41) for receiving Kirschner wires (42) are provided in the retaining bars (2) and/or near the point of intersection (7) of the scissors assembly (3-7) and/or at at least one end (29) of the actuating instrument (25).

10. A retractor according to Claim 9, **characterised in that** the bores (41) for the Kirschner wires (42) are also disposed near the blade couplings (10) and the blades (11) also include bores (41) for receiving Kirschner wires (42).

## Revendications

1. Ecarteur chirurgical comportant des valves (11) qui sont disposées sur des rails de maintien (2), qui sont dotés d'un guidage parallèle (3-7) et d'un verrouillage de rappel (21, 22), **caractérisé en ce que** l'écarteur est relié ou peut être relié, par des couplages d'actionnement (10, 19) détachables à l'état écarté, avec un instrument d'actionnement (25) en forme de ciseau.

2. Ecarteur selon la revendication 1, **caractérisé en ce qu'**un dispositif en ciseau (3-7) est prévu comme guidage parallèle des rails de maintien (2).

3. Ecarteur selon la revendication 2, **caractérisé en ce qu'**une valve médiane (17) ou un rail de maintien de valve (16) est disposé sur le point de croisement (7) du dispositif en ciseau (3-7).

4. Ecarteur selon la revendication 3, **caractérisé en ce que** le rail médian de maintien de valve (16) est doté d'un couplage d'actionnement (19) pour la liaison avec un élément (3) de l'instrument d'actionnement (25) et celui-ci porte sur l'autre élément (3) une valve (17) ou un couplage de valve (10).

5. Ecarteur selon l'une quelconque des revendications 1 à 4, **caractérisé en ce que** les couplages d'actionnement (10, 19) et les couplages de valves (10, 19) sont réalisés de façon concordante.

6. Ecarteur selon l'une quelconque des revendications 3 à 5, **caractérisé en ce que**, outre le guidage parallèle (3-7), l'instrument d'actionnement (25) est également doté d'un verrouillage de rappel (30).

7. Ecarteur selon l'une quelconque des revendications 1 à 6, **caractérisé en ce que** les rails de maintien (2) guidés parallèlement présentent respectivement une rangée de couplages de valves (10).

8. Ecarteur selon la revendication 7, **caractérisé en ce qu'**un ou plusieurs instruments supplémentaires (35) sont dotés de couplages adaptés aux couplages de valves (10) des rails de maintien (2).

9. Ecarteur selon l'une quelconque des revendications 3 à 8, **caractérisé en ce que**, dans les rails de maintien (2) et/ou près du point de croisement (7) du dispositif en ciseau (3-7) et/ou sur au moins une extrémité (29) de l'instrument d'actionnement (25), des perçages (41) sont prévus pour recevoir des fils de Kirschner (42).

10. Ecarteur selon la revendication 9, **caractérisé en ce que** les perçages (41) pour les fils de Kirschner (42) sont également disposés près des couplages de valves (10) et **en ce que** les valves (11) comprennent également des perçages (41) pour recevoir des fils de Kirschner (42).
